# FASCICULE DE BREVET EUROPEEN

(11) **EP 0 869 753 B1**
(45) Date de publication et mention de la délivrance du brevet: **05.07.2000**
(21) Numéro de dépôt: 96942435.7
(22) Date de dépôt: 19.12.1996
(51) Int. Cl.: A61F 2/44

(54) **CAGE VERTEBRALE INTERSOMATIQUE**
ZWISCHENWIRBELKÖRPER
INTERBODY VERTEBRAL IMPLANT

(30) Priorité: 21.12.1995 FR 9515601
(43) Date de publication de la demande: 14.10.1998
(73) Titulaire: Colorado, 69230 Saint-Genis-Laval (FR)
(72) Inventeur: GROSSE, Arsène, F-67400 Illkirch-Graffenstaden (FR); BRAUN, Emmanuel, F-54000 Nancy (FR); DEHOUX, Emile, F-05100 Briançon (FR); DELEFORTRIE, Guido, B-5140 Ligny (BE); MUNTING, Everard, B-1390 Piez (BE)
(74) Mandataire: Martin, Jean-Paul
(86) Numéro de dépôt international: FR9602033
(87) Numéro de publication internationale: WO9723175

(56) Documents cités:
- EP-A- 0 307 241
- WO-A-91/13598
- DE-A- 4 328 690
- FR-A- 2 703 580
- US-A- 2 677 369

## Description

La présente invention concerne une cage vertébrale intersomatique, destinée à être implantée par voie d'abord antérieure ou latérale, pour réaliser l'immobilisation de deux vertèbres adjacentes. Cette cage conforme au préambule de la revendication 1, est notamment destinée à réaliser l'immobilisation des quatrième et cinquième vertèbres lombaires, ou de la cinquième vertèbre lombaire et de la première vertèbre du sacrum.

Un disque intervertébral peut s'affaisser à la longue, provoquant une compression locale de la moelle épinière et des racines nerveuses. Il est alors nécessaire d'insérer un implant osseux entre les plateaux des deux vertèbres concernées, pour rétablir l'espace intervertébral anatomique et pour immobiliser les deux vertèbres, par ostéogenèse.

Une technique consiste à implanter un greffon osseux directement entre les vertèbres, après ablation du disque. Ce greffon peut être constitué par un tronçon diaphysaire d'os long, bourré d'os spongieux.

Il apparaît toutefois qu'un tel greffon résiste mal aux contraintes générées par les mouvements du patient et présente un risque important d'insertion à la longue dans l'une ou l'autre des vertèbres.

Pour remédier à cet inconvénient, il a été envisagé de placer le greffon osseux dans un implant rigide, ou "cage intersomatique", inséré entre les vertèbres, cette cage étant ouverte à ses extrémités supérieure ou inférieure pour permettre la venue du greffon au contact de l'os spongieux des plateaux vertébraux.

De telles cages peuvent être implantées par voie d'abord postérieure. La présence de la moelle épinière et des racines nerveuses oblige alors à l'implantation de deux cages sensiblement parallèles, disposées de part et d'autre de l'axe du rachis, afin d'assurer la stabilisation et la bonne fusion des vertèbres.

Ainsi le FR-A-2.703.580 décrit une cage intervertébrale en polyéthylène, ayant un contour fermé et une cloison intérieure, des crans étant agencés sur le pourtour des faces opposées. Ces crans superficiels sont insuffisants pour assurer un guidage et un ancrage convenables de la cage.

Le EP-A-0.307.241 concerne une cage intervertébrale de petites dimensions, munie de nervures superficielles. Deux cages de ce genre doivent être disposées entre les vertèbres par la voie postérieure, et non antérieure ou latérale.

Le DE-43.28.690 a trait à un implant intervertébral en U qui ne constitue pas une cage proprement dite, c'est à dire ayant un contour fermé et des ouvertures latérales. Cet implant est pourvu de languettes superficielles de guidage et de maintien dans le plan sagittal.

Dans certains cas, il est nécessaire ou préférable d'implanter une cage unique destinée à occuper la majeure partie de l'espace intervertébral. Une telle cage implique une implantation par voie antérieure ou latérale.

Les cages existantes s'avèrent difficiles à insérer par voie antérieure et à positionner entre les vertèbres, en particulier entre les quatrième et cinquième vertèbres lombaires ou entre les vertèbres situées au-dessus, compte tenu de leurs dimensions importantes.

La présente invention vise à remédier à cet inconvénient en fournissant une cage implantable par voie d'abord antérieure ou latérale, selon le niveau vertébral concerné, qui soit facile et rapide à implanter, et qui soit parfaitement maintenue par rapport aux vertèbres.

Selon l'invention, la cage comprend au moins deux parois faisant saillie de ses faces supérieure et inférieure, parallèlement à la direction d'introduction de la cage entre les vertèbres, ces parois étant conformées pour s'insérer progressivement dans l'os spongieux lors de cette introduction puis pour immobiliser la cage dans sa position d'implantation, le temps que des moyens d'ancrage de la cage à l'une des vertèbres soient mis en place.

Ces parois saillantes permettent ainsi de guider la cage vers sa position d'implantation, puis de l'immobiliser lorsque cette position est atteinte, ce qui facilite la mise en place ultérieure des moyens d'ancrage définitifs de la cage à l'une des vertèbres.

Une fois l'ostéogenèse opérée, ces parois insérées dans l'os spongieux constituent des moyens de stabilisation des vertèbres par rapport à la cage.

De préférence, la cage comprend deux parois supérieures et deux parois inférieures saillantes telles que précitées, qui assurent un parfait guidage de la cage en translation lors de son introduction entre les vertèbres.

Selon une forme de réalisation préférée de l'invention, chaque paroi en saillie présente une partie antérieure, par rapport au sens d'insertion de la cage, qui augmente progressivement en hauteur, et une partie postérieure se terminant par un bord relativement abrupt, qui réalise un certain "verrouillage" de la cage lorsque celle-ci atteint sa position d'implantation entre les vertèbres.

De préférence, la partie antérieure de chaque paroi en saillie est tranchante et/ou présente des aspérités permettant d'entailler l'os spongieux pour faciliter l'introduction de la cage, alors que la partie postérieure de ces saillies présente une surface non agressive, propre à former une butée permettant ledit "verrouillage".

Avantageusement, deux parois faisant saillie de deux faces opposées de la cage sont reliées l'une à l'autre par une cloison intermédiaire, ce qui assure une bonne homogénéité de structure à la cage.

De préférence, les moyens d'ancrage définitif de la cage à l'une des vertèbres sont constitués par une vis engagée au travers d'un trou aménagé dans la paroi latérale de la cage.

Avantageusement, ce trou présente une hauteur telle qu'il permet un débattement de la vis dans un plan vertical, de telle sorte que cette vis puisse être orientée vers l'une ou l'autre des vertèbres, au choix du praticien.

De préférence, une cavité hémisphérique est aménagée dans la paroi latérale de la cage, en face du trou recevant la vis d'ancrage, et la vis employée présente une tête dont la paroi périphérique a la forme d'un segment de sphère. Cette cavité permet de loger la tête de la vis, qui ne fait donc pas saillie au-delà de la paroi latérale de la cage, et permet de faciliter l'orientation de la vis vers l'une ou l'autre des vertèbres.

Selon une forme préférée de réalisation de l'invention, la largeur du trou recevant la vis est inférieure au diamètre extérieur du filet de cette vis, et la vis comprend une portée cylindrique intermédiaire située entre sa tête et son corps fileté, cette portée étant située à hauteur du trou lorsque la vis est implantée et permettant un blocage axial de la vis. Ce blocage élimine tout risque de déplacement axial de la vis sous l'effet des contraintes répétées exercées sur la cage par les mouvements du patient.

Selon une forme de réalisation préférée de l'invention, la cage comprend deux cloisons parallèles et le trou recevant la vis est aménagé entre ces deux cloisons.

Pour sa bonne compréhension, l'invention est à nouveau décrite ci-dessous en référence au dessin schématique annexé représentant, à titre d'exemples non limitatifs, deux formes de réalisation préférées de la cage vertébrale intersomatique qu'elle concerne.
La figure 1 est une vue en perspective d'une cage destinée à être implantée par voie antérieure ;
la figure 2 est une vue de profil, partiellement en coupe, d'une cage conforme à la figure 1, destinée à l'immobilisation des quatrième et cinquième vertèbres lombaires, après implantation ;
la figure 3 est une vue de profil d'une cage conforme à la figure 1, destinée à l'immobilisation de la cinquième vertèbre lombaire et de la première vertèbre du sacrum, après implantation ;
la figure 4 est une vue en perspective d'une cage destinée à être implantée par voie latérale, et
les figures 5 et 6 sont des vues de cette cage respectivement de profil et de face, après implantation entre deux vertèbres.

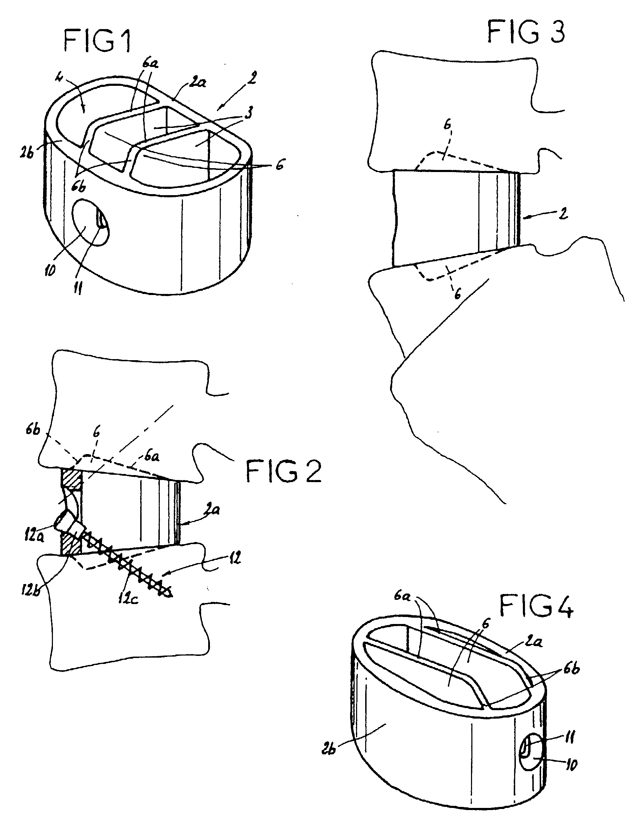

La figure 1 représente une cage inter-vertébrale 2, destinée à être implantée par voie d'abord antérieure entre deux vertèbres adjacentes pour réaliser l'immobilisation de ces vertèbres.

Cette cage comprend deux cloisons transversales 3, orientées selon une direction antéro-postérieure, c'est-à-dire parallèlement à la direction d'introduction de la cage entre les vertèbres. Ces cloisons 3 délimitent trois cavités 4 de réception de greffons ou de copeaux d'os spongieux, qui sont ouvertes à leurs extrémités supérieure et inférieure. Ces ouvertures permettent la venue des greffons ou copeaux au contact de l'os spongieux des plateaux vertébraux afin que la fusion de la cage avec ces plateaux puisse s'opérer, par ostéogenèse.

La cage 2 présente une forme sensiblement annulaire, avec une face postérieure 2a rectiligne et une face antérieure 2b courbe, de forme correspondant sensiblement à la courbure antérieure des plateaux vertébraux.

Ainsi que cela apparaît sur les figures 2 et 3, cette cage 2 est dimensionnée de manière à occuper la majeure partie de l'espace intervertébral.

La figure 2 montre une cage 2 destinée à être implantée entre les quatrième et cinquième vertèbres lombaires. Dans ce cas, la cage présente des faces supérieure et inférieure qui convergent en direction de sa face postérieure 2a, de manière symétrique par rapport à un plan médian horizontal, en formant l'une par rapport à l'autre un angle de l'ordre de 10 degrés.

Par contre, comme le montre la figure 3, la cage 2 destinée à être implantée entre la cinquième vertèbre lombaire et la première vertèbre du sacrum présente une face supérieure sensiblement horizontale et une face inférieure inclinée par rapport à cette face supérieure, selon un angle de l'ordre de 10 degrés.

Il apparaît en outre sur les figures que les cloisons 3 font saillie au-delà des faces supérieure et inférieure de la cage 2, et délimitent ainsi des parois saillantes 6.

Ces parois 6 ont une forme en "aileron", c'est-à-dire comprennent une partie antérieure 6a, par rapport au sens d'insertion de la cage, qui augmente progressivement en hauteur, et une partie postérieure se terminant par un bord 6b relativement abrupt, situé en retrait de la face antérieure 2b de la cage 2.

En outre, la cage 1 comprend une cavité hémisphérique 10 aménagée dans sa face antérieure 2b, débouchant dans la cavité 4 centrale par un trou 11.

Cette cavité hémisphérique 10 et ce trou 11 sont destinés à recevoir une vis d'ancrage osseux 12, représentée à la figure 2.

La vis 12 présente une tête 12a dont la paroi périphérique a une forme en segment de sphère, et une portée cylindrique 12b située entre la tête 12a et le corps fileté 12c de cette vis.

Le trou 11 est oblong. Sa longueur est orientée parallèlement aux cloisons 3 et est telle qu'elle permet un débattement de la vis 12 dans un plan vertical. La largeur du trou 11 est inférieure au diamètre extérieur du filet du corps 12c mais légèrement supérieure au diamètre de la portée cylindrique 12b.

Comme le montrent les figures 2 et 3, lors de l'introduction de la cage 2 entre les vertèbres, les parois 6 viennent s'insérer progressivement dans l'os spongieux des plateaux vertébraux, grâce à l'augmentation progressive de leur hauteur au niveau de leur parties antérieures 6a.

Les quatre parois 6 supérieures et inférieures assurent un parfait guidage de la cage 2 en translation lors de cette introduction.

Lorsque la cage 2 atteint sa position d'implantation, les bords postérieurs 6b sont également insérés dans l'os spongieux. Par leur forme relativement abrupte, ces bords constituent des butées réalisant un certain "verrouillage" de la cage 2 dans cette position d'implantation, le temps que la vis 12 soit mise en place.

Le débattement de cette vis 12 dans un plan vertical, rendu possible par le trou 11, permet l'ancrage de la vis dans l'une ou l'autre des vertèbres, au choix du praticien.

La cavité hémisphérique 10 facilite l'orientation de la vis 12, et permet de loger la tête 12a, qui ne fait que peu saillie au-delà de la face 2b.

La largeur du trou 11 inférieure au diamètre externe du filet permet d'immobiliser axialement la vis 12 lorsque celle-ci est serrée, afin d'éliminer tout risque de déplacement axial de la vis sous l'effet des contraintes répétées exercées sur la cage par les mouvements du patient.

Une fois la fusion opérée, les parois 6 constituent des moyens de stabilisation des vertèbres par rapport à la cage 2.

Les cloisons 3 assurent, quant à elles, une bonne homogénéité de structure à la cage 2.

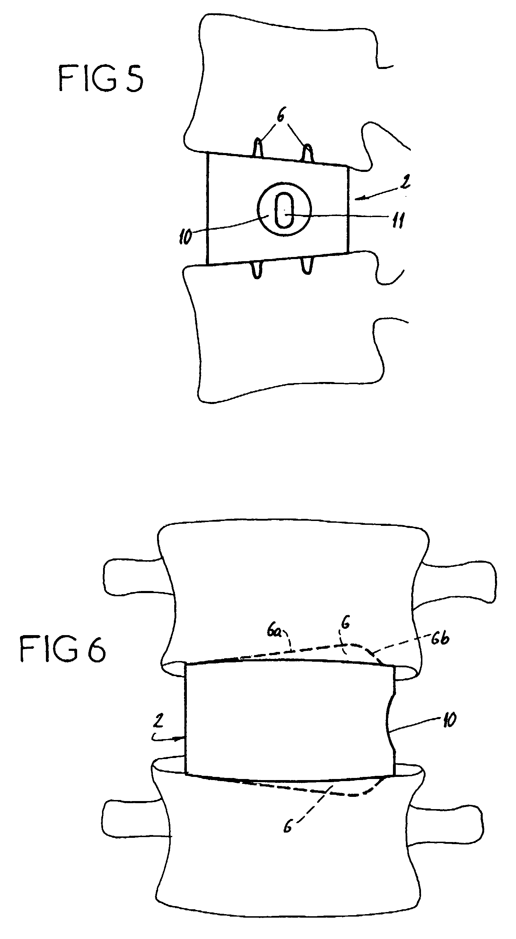

Les figures 4 à 6 montrent une cage destinée à être implantée par voie d'abord latérale. Par simplification, les éléments déjà décrits en référence aux figures 1 à 3 qui se retrouvent dans cette cage sont désignés par les mêmes références numériques.

Cette cage est en tous points identique à celles représentées aux figures 1 à 3, sinon que les cloisons 3 et les parois 6 sont orientées dans le sens de la longueur de la cage, c'est-à-dire, ici également, parallèlement à la direction d'introduction de la cage 2 entre les vertèbres.

Il va de soi que l'invention n'est pas limitée aux formes de réalisation décrites ci-dessus à titre d'exemples mais qu'elle en embrasse, au contraire, toutes les variantes de réalisation. Ainsi, la cage pourrait comprendre une seule paroi 6 supérieure et une seule paroi 6 inférieure.

## Revendications

1. Cage intersomatique (2) destinée à être implantée entre deux vertèbres adjacentes par voie antérieure, présentant une forme sensiblement annulaire avec une face postérieure (2a) et une face antérieure (2b), caractérisée en ce qu'elle comporte des cloisons (3) délimitant des cavités (4) de réception de greffons ou de copeaux d'os spongieux, en ce que ces cloisons font saillie au-delà des faces supérieure et inférieure de la cage en délimitant ainsi des parois saillantes(6) parallèles à la direction antéro-postérieure d'introduction de la cage entre les vertèbres, et en ce que chaque paroi saillante (6) présente une partie antérieure (6a) par rapport au sens d'insertion de la cage (2), qui augmente progressivement en hauteur, et une partie postérieure se terminant par un bord (6b) relativement abrupt, qui assure un verrouillage de la cage (2) lorsque celle-ci atteint sa position d'implantation entre les vertèbres.

2. Cage intersomatique (2) destinée à être implantée entre deux vertèbres adjacentes par voie latérale, présentant une forme sensiblement annulaire avec une face postérieure (2a) et une face antérieure (2b), caractérisée en ce qu'elle comporte des cloisons (3) délimitant des cavités (4) de réception de greffons ou de copeaux d'os spongieux, en ce que ces cloisons font saillie au-delà des faces supérieure et inférieure de la cage en délimitant ainsi des parois saillantes (6) parallèles à la direction latérale d'introduction de la cage entre les vertèbres, et en ce que chaque paroi saillante (6) présente une partie antérieure (6a) par rapport au sens d'insertion de la cage (2), qui augmente progressivement en hauteur, et une partie postérieure se terminant par un bord (6b) relativement abrupt, qui assure un verrouillage de la cage (2) lorsque celle-ci atteint sa position d'implantation entre les vertèbres.

3. Cage selon la revendication 1 ou 2, caractérisée en ce qu'elle comprend deux parois (6) supérieures et deux parois (6) inférieures.

4. Cage selon la revendication 1 ou 2, caractérisée en ce que la partie antérieure (6a) de chaque paroi en saillie (6) est tranchante et/ou présente des aspérités permettant d'entailler l'os pour faciliter l'introduction de la cage entre les vertèbres, alors que la partie postérieure (6b) de ces parois (6) présente une surface non agressive, propre à former une butée permettant ledit verrouillage.

5. Cage selon l'une des revendications 1 à 3, caractérisée en ce que deux parois (6) faisant saillie de deux de ses faces opposées sont reliées l'une à l'autre par une cloison intermédiaire (3).

6. Cage selon l'une des revendications 1 à 5, caractérisée en ce que ses moyens d'ancrage définitif à l'une des vertèbres sont constitués par une vis (12) engagée au travers d'un trou (11) aménagé dans sa paroi latérale (2b).

7. Cage selon la revendication 6, caractérisée en ce que le trou (11) présente une hauteur telle qu'il permet un débattement de la vis (12) dans un plan vertical, de telle sorte que cette vis (12) puisse être orientée vers l'une ou l'autre des vertèbres.

8. Cage selon les revendications 6 ou 7, caractérisée en ce qu'une cavité hémisphérique (10) est aménagée dans sa paroi latérale (2b), en face du trou (11) recevant la vis d'ancrage (12), et en ce que la vis (12) présente une tête (12a) dont la paroi périphérique a la forme d'un segment de sphère.

9. Cage selon l'une des revendications 6 à 8, caractérisée en ce que la largeur du trou (11) recevant la vis (12) est inférieure au diamètre extérieur du filet de cette vis (12), et en ce que la vis (12) comprend une portée cylindrique intermédiaire (12b) située entre sa tête (12a) et son corps fileté (12c), cette portée (12b) étant située à hauteur du trou (11) lorsque la vis (12) est implantée et permettant un blocage axial de cette vis (12).

10. Cage selon l'une des revendications 6 à 9, caractérisée en ce qu'elle comprend deux cloisons parallèles (3) et en ce que le trou (11) recevant la vis (12) est aménagé entre ces deux cloisons (3).

11. Cage selon l'une des revendications 1 à 10, destinée à être implantée entre les quatrième et cinquième vertèbres lombaires, caractérisée en ce qu'elle présente des faces supérieure et inférieure qui convergent en direction de sa face postérieure (2a), de manière symétrique par rapport à un plan médian horizontal, en formant l'une par rapport à l'autre un angle de l'ordre de 10 degrés.

12. Cage selon l'une des revendications 1 à 10, destinée à être implantée entre la cinquième vertèbre lombaire et la première vertèbre du sacrum, caractérisée en ce qu'elle présente une face supérieure sensiblement horizontale et une face inférieure inclinée par rapport à cette face supérieure, selon un angle de l'ordre de 10 degrés.

## Patentansprüche

1. Zwischenwirbelkörper (2), der dazu bestimmt ist, von vorn zwischen zwei benachbarte Wirbel implantiert zu werden, mit einer im wesentlichen ringförmigen Gestalt mit einer Vorderseite (2a) und einer Rückseite (2b), dadurch **gekennzeichnet**, daß er Zwischenwände (3) aufweist, die Hohlräume (4) zur Aufnahme von Körnern oder Schnitzeln aus schwammförmigem Knochengewebe begrenzen, daß diese Zwischenwände über die oberen und unteren Oberflächen des Körpers hinaus vorspringen und somit vorspringende Wände parallel zur Frontal/Dorsal-Einführungsrichtung des Körpers zwischen die Wirbel bilden, und daß jede vorspringende Wand (6) einen in Bezug auf die Einführungsrichtung des Körpers (2) vorderen Teil (6a), dessen Höhe progressiv zunimmt, und einen rückwärtigen Teil hat, der in einem relativ steilen Rand (6b) endet, der eine Verriegelung des Körpers (2) gewährleistet, wenn dieser seine Implantationsstellung zwischen den Wirbeln erreicht.

2. Zwischenwirbelkörper (2), der dazu bestimmt ist, von der Seite her zwischen zwei benachbarte Wirbel implantiert zu werden, mit einer im wesentlichen ringförmigen Gestalt mit einer Vorderseite (2a) und einer Rückseite (2b), dadurch **gekennzeichnet**, daß er Zwischenwände (3) aufweist, die Hohlräume (4) zur Aufnahme von Körnern oder Schnitzeln aus schwammförmigem Knochengewebe begrenzen, daß diese Zwischenwände über die oberen und unteren Oberflächen des Körpers hinaus vorspringen und somit vorspringende Wände (6) parallel zur Seitwärts-Einführungsrichtung zwischen die Wirbel bilden, und daß jede vorspringende Wand (6) einen in Bezug auf die Einführungsrichtung des Körpers (2) vorderen Teil (6a), dessen Höhe progressiv zunimmt, und einen rückwärtigen Teil hat, der in einem relativ steilen Rand (6b) endet, der eine Verriegelung des Körpers (2) gewährleistet, wenn dieser seine Implantationsstellung zwischen den Wirbeln erreicht.

3. Körper nach Anspruch 1 oder 2, dadurch **gekennzeichnet**, daß er zwei obere Wände (6) und zwei untere Wände (6) aufweist.

4. Körper nach Anspruch 1 oder 2, dadurch **gekennzeichnet**, daß der vordere Teil (6a) jeder vorspringenden Wand (6) schneidend ist und/oder Unebenheiten aufweist, die es gestatten in den Knochen einzuschneiden, um das Einführen des Körpers zwischen die Wirbel zu erleichtern, während der rückwärtige Teil (6b) dieser Wände (6) eine nicht aggressive Oberfläche bildet, die dazu geeignet ist, einen Anschlag zu bilden, der die genannte Verriegelung ermöglicht.

5. Körper nach einem der Ansprüche 1 bis 3, dadurch **gekennzeichnet**, daß zwei Wände (6), die von seinen beiden entgegengesetzten Oberflächen vorspringen, durch eine Zwischenwand (3) miteinander verbunden sind.

6. Körper nach einem der Ansprüche 1 bis 5, dadurch **gekennzeichnet**, daß seine Mittel zur endgültigen Verankerung an einem der Wirbel durch eine Schraube (12) gebildet werden, die durch ein in seiner Seitenwand (2b) ausgebildetes Loch (11) gesteckt ist.

7. Körper nach Anspruch 6, dadurch **gekennzeichnet**, daß das Loch (11) eine Höhe hat, die eine Auslenkung der Schraube (12) in einer vertikalen Ebene ermöglicht, derart, daß diese Schraube (12) auf den einen oder den anderen der Wirbel gerichtet werden kann.

8. Körper nach Anspruch 6 oder 7, dadurch **gekennzeichnet**, daß in seiner Seitenwand (2b), die dem Loch (11) zugewandt ist, das die Verankerungsschraube (12) aufnimmt, eine halbkugelige Höhlung (10) ausgebildet ist und daß die Schraube (12) einen Kopf (12a) hat, dessen Umfangswand die Form eines Kugelsegments hat.

9. Körper nach einem der Ansprüche 6 bis 8, dadurch **gekennzeichnet**, daß die Breite des Loches (11), das die Schraube (12) aufnimmt, kleiner ist als der Außendurchmesser des Gewindes dieser Schraube (12) und daß die Schraube (12) einen zylindrischen Zwischenabschnitt (12b) zwischen ihrem Kopf (12a) und dem Gewindeabschnitt (12c) aufweist, wobei dieser Zwischenabschnitt (12b) in Höhe des Loches (11) liegt, wenn die Schraube (12) implantiert ist, und es gestattet, diese Schraube (12) in Axialrichtung zu blockieren.

10. Körper nach einem der Ansprüche 6 bis 9, dadurch **gekennzeichnet**, daß er zwei parallele Zwischenwände (3) aufweist, und daß das Loch (11), das die Schraube (12) aufnimmt, zwischen den beiden Zwischenwänden (3) ausgebildet ist.

11. Körper nach einem der Ansprüche 1 bis 10, der dazu bestimmt ist, zwischen dem vierten und fünften Lendenwirbel implantiert zu werden, dadurch **gekennzeichnet**, daß er obere und untere Oberflächen aufweist, die symmetrisch zu einer horizontalen Medianebene in Richtung auf seine Rückseite (2a) konvergieren und miteinander einen Winkel in der Größenordnung von 10° bilden.

12. Körper nach einem der Ansprüche 1 bis 10, der dazu bestimmt ist, zwischen dem fünften Lendenwirbel und dem ersten Kreuzbeinwirbel implantiert zu werden, dadurch **gekennzeichnet**, daß er eine im wesentlichen waagerechte obere Oberfläche und eine untere Oberfläche bildet, die in Bezug auf diese obere Oberfläche unter einem Winkel in der Größenordnung von 10° geneigt ist.

## Claims

1. Interbody cage (2) intended to be implanted between two adjacent vertebrae by the anterior route, having a substantially annular shape with a posterior face (2a) and an anterior face (2b), characterised in that it comprises partitions (3) defining cavities (4) for receiving grafts or chips of spongy bone, in that these partitions project beyond the upper and lower surfaces of the cage, thus defining projecting walls (6) parallel to the front-to-back direction of insertion of the cage between the vertebrae, and in that each projecting wall (6) has an anterior portion (6a) relative to the direction of insertion of the cage (2), which increases progressively in height, and a posterior portion terminating in a relatively abrupt edge (6b), which ensures that the cage (2) is locked when it reaches its position of implantation between the vertebrae.

2. Interbody cage (2) intended to be implanted between two adjacent vertebrae by the lateral route, having a substantially annular shape with a posterior face (2a) and an anterior face (2b), characterised in that it comprises partitions (3) defining cavities (4) for receiving grafts or chips of spongy bone, in that these partitions project beyond the upper and lower surfaces of the cage, thus defining projecting walls (6) parallel to the lateral direction of insertion of the cage between the vertebrae, and in that each projecting wall (6) has an anterior portion (6a) relative to the direction of insertion of the cage (2), which increases progressively in height, and a posterior portion terminating in a relatively abrupt edge (6b), which ensures that the cage (2) is locked when it reaches its position of implantation between the vertebrae.

3. Cage according to claim 1 or 2, characterised in that it comprises two upper walls (6) and two lower walls (6).

4. Cage according to claim 1 or 2, characterised in that the anterior portion (6a) of each projecting wall (6) is a cutting edge and/or has roughened parts enabling the bone to be notched in order to aid the insertion of the cage between the vertebrae, whilst the posterior portion (6b) of these walls (6) has a non-aggressive surface adapted to form an abutment allowing said locking to take place.

5. Cage according to one of claims 1 to 3, characterised in that two walls (6) projecting from two of the opposing surfaces thereof are connected to one another by an intermediate partition (3).

6. Cage according to one of claims 1 to 5, characterised in that its means for permanent anchoring to one of the vertebrae consist of a screw (12) passing through a hole (11) provided in the side wall (2b).

7. Cage according to claim 6, characterised in that the hole (11) is of a height such that it allows the screw (12) to move in a vertical plane, so that this screw (12) can be oriented towards one or other of the vertebrae.

8. Cage according to claim 6 or 7, characterised in that a hemispherical cavity (10) is provided in its side wall (2b), opposite the hole (11) which accommodates the anchoring screw (12), and in that the screw (12) has a head (12a) the peripheral wall of which is in the shape of a spherical segment.

9. Cage according to one of claims 6 to 8, characterised in that the width of the hole (11) accommodating the screw (12) is less than the outer diameter of the thread of this screw (12), and in that the screw (12) comprises an intermediate cylindrical bearing surface (12b) located between its head (12a) and its threaded body (12c), this bearing surface (12b) being located at the height of the hole (11) when the screw (12) is in place and permitting axial securing of this screw (12).

10. Cage according to one of claims 6 to 9, characterised in that it comprises two parallel partitions (3) and in that the hole (11) which accommodates the screw (12) is provided between these two partitions (3).

11. Cage according to one of claims 1 to 10, intended to be implanted between the fourth and fifth lumbar vertebrae, characterised in that it has upper and lower surfaces which converge towards its posterior face (2a), symmetrically with respect to a horizontal median plane, forming an angle of the order of 10 degrees relative to one another.

12. Cage according to one of claims 1 to 10, intended to be implanted between the fifth lumbar vertebra and the first vertebra of the sacrum, characterised in that it has a substantially horizontal upper surface and a lower surface which is inclined relative to this upper surface at an angle of the order of 10 degrees.
